# EUROPEAN PATENT APPLICATION

(11) **EP 3 222 258 A1**
(43) Date of publication of application: **27.09.2017**
(21) Application number: 16174837.1
(22) Date of filing: 16.06.2016
(51) Int. Cl.: A61F 13/472

(54) **SANITARY PAD**

(30) Priority: 23.03.2016 CN 201610167739
(71) Applicant: Chien, Yuan-Cheng, Kaohsiung City (TW)
(72) Inventor: Chien, Yuan-Cheng, Kaohsiung City (TW)
(74) Representative: Gulde & Partner

(57) **Abstract**

Provided is a sanitary pad which comprises a main body (10) and a raised flow-guiding element (20). The raised flow-guiding element (20) comprises a flow-guiding layer (21), a front sheet (22), a back sheet (23) and multiple perforations (24). Each of the two lateral sides of the flow-guiding layer (21) is respectively and intermittently connected to the main body (10). The maximum distance between a leading edge of the flow-guiding layer (21) and the main body (10) is smaller than that between a trailing edge of the flow-guiding layer (21) and the main body (10). Each of the multiple perforations (24) is formed on the top of the flow-guiding layer (21). The front sheet (22) is extended from the leading edge of the flow-guiding layer (21) and connected to the main body (10). The back sheet (23) is extended from the trailing edge of the flow-guiding layer (21).

## Description

The present invention relates to a sanitary pad, especially to the sanitary pad with a raised flow-guiding element.

Taiwan issued Patent No. I466663 discloses a conventional sanitary napkin. The conventional sanitary napkin comprises an absorption layer, and the absorption layer comprises a diversion layer, and the diversion layer is positioned on the absorption layer. The diversion layer comprises a central part. Each of the two lateral sides of the central part is respectively extended to the absorption layer and forms a lateral wall. A front end of the central part is not connected to the two lateral walls and the absorption layer. A back end of the central part is extended to form a pull portion. The pull portion is extended beyond the absorption layer.

As a user uses the conventional sanitary napkin, the front end of the central part covers the user's labia majora and surrounds the labia minora of the user. The pull portion of the central part can be pulled by the user and covered on the user's buttock.

However, for the reason that the front end of the central part is not connected to the two lateral walls and the absorption layer, the front end of the central part is planar. The front end of the central part cannot be inserted into and adhered to the user's labia minora, causing the poor adhesion effect, low absorption and low flow-guiding efficiency of menstrual blood.

The present invention provides a sanitary pad to mitigate or obviate the aforementioned problems.

The sanitary pad in accordance with the present invention has a main body and a raised flow-guiding element. The main body comprises an absorption layer and a laminated edge. The laminated edge is arranged surrounding the main body. The raised flow-guiding element is positioned on the top of the absorption layer of the main body. The raised flow-guiding element comprises an arch-shaped flow-guiding layer, a front sheet, a back sheet, and multiple perforations. Each of the two lateral sides of the flow-guiding layer is respectively and intermittently connected to the main body. The maximum distance between a leading edge of the flow-guiding layer and the main body is smaller than the maximum distance between a trailing edge of the flow-guiding layer and the main body. The front sheet is extended from the leading edge of the flow-guiding layer, and a leading edge of the front sheet is connected to the main body. The back sheet is extended from the trailing edge of the flow-guiding layer, and the back sheet is extended beyond the laminated edge of the main body. Each of the multiple perforations is formed on the top of the flow-guiding layer.

Preferably, the sanitary pad further comprises a ventilation unit. The ventilation comprises multiple holes. The ventilation unit is positioned on a surface of the raised flow-guiding element and covers the flow-guiding layer, allowing an interspace to be formed between the ventilation unit and the flow-guiding layer of the raised flow-guiding element. Each of the multiple holes is distributed on the ventilation unit, and the interspace is connected to each of the multiple holes of the ventilation unit and each of the multiple perforations.

Preferably, the ventilation unit further comprises a sheet. Each of the two lateral sides of the sheet is respectively connected to each of the two lateral sides of the flow-guiding layer. The sheet is positioned adjacent to the leading edge of the flow-guiding layer. Each of the multiple holes is distributed on the sheet of the ventilation unit.

Preferably, the ventilation unit further comprises multiple sheets. Each of the multiple sheets is positioned intermittently on the raised flow-guiding element. Each of the two lateral sides of each of the sheets is respectively connected to each of the two lateral sides of the flow-guiding layer. Each of the multiple holes is distributed on each of the multiple sheets.

Preferably, two lateral sides of the front sheet are not connected to the main body.

Preferably, the maximum distance between a leading edge of the flow-guiding layer and the main body is smaller than the maximum distance between a trailing edge of the flow-guiding layer and the main body.

Preferably, the maximum distance between a leading edge of the flow-guiding layer and the main body is smaller than the maximum distance between a trailing edge of the flow-guiding layer and the main body.

As a user uses the sanitary pad in accordance with the present invention, the user can pull the back sheet of the raised flow-guiding element, allowing a top of the flow-guiding layer and the front sheet of the raised flow-guiding element to be inserted and adhered to the user's labia minora and the flow-guiding layer adjacent to the absorption layer of the main body to be adhered to the user's labia majora. The flow-guiding layer and the front sheet of the raised flow-guiding element can absorb the menstrual blood flowing to the labia minora directly and quickly, followed by guiding the absorbed menstrual blood to the main body, rather than absorbing the menstrual blood when the blood flows out of the labia minora. Therefore, the raised flow-guiding element prevents the menstrual blood from overflowing from the user's perineum and intergluteal cleft. Besides, the multiple perforations formed on the top of the flow-guiding layer enhance absorption efficiency of menstrual blood. Moreover, the interspace formed between the sheet of the ventilation unit and the flow-guiding layer of the raised flow-guiding element enhances breathability and comfort. Furthermore, the back sheet extended beyond the laminated edge of the main body is convenient to the user to pull and adjust, allowing the back sheet to be placed between the user and the main body.

### In the drawings:

Fig. 1 is a top view of a first embodiment of a sanitary pad in accordance with the present invention;
Fig. 2 is a side view of the first embodiment of the sanitary pad in Fig. 1;
Fig. 3 is cross-sectional view of the first embodiment of the sanitary pad across line A-A in Fig. 2;
Fig. 4 is a side view of the second embodiment of the sanitary pad in accordance with the present invention;
Fig. 5 is a side view of the third embodiment of the sanitary pad in accordance with the present invention; and,
Fig. 6 is a side-sectional view of the sanitary pad in a usage state in Fig. 1.

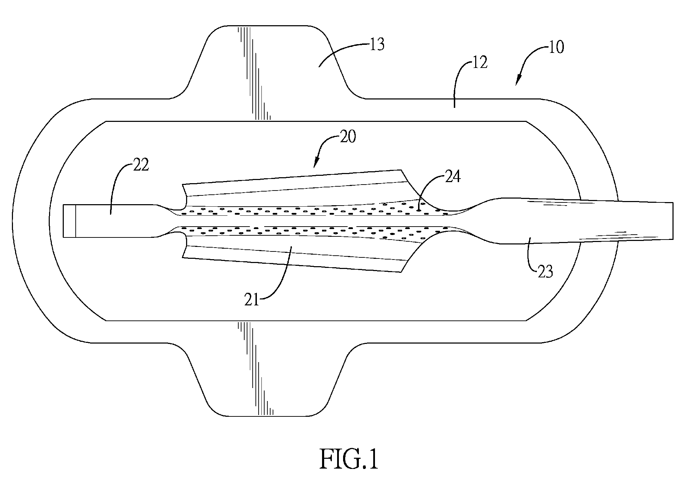

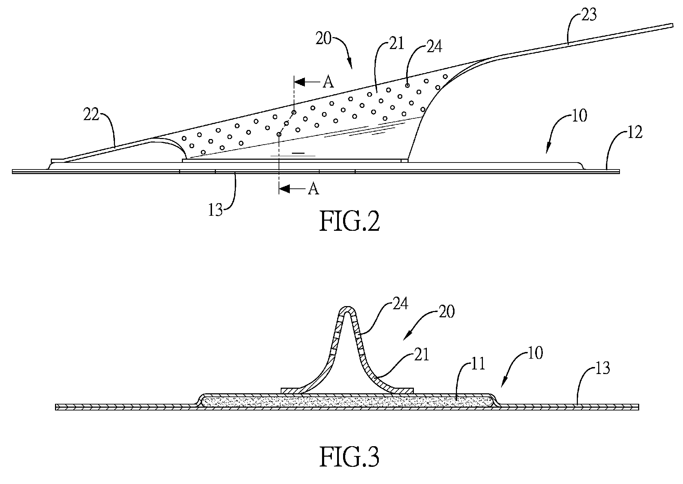

With references to Figs. 1 to 3, a sanitary pad of a first embodiment in accordance with the present invention comprises a main body 10 and a raised flow-guiding element 20.

The main body 10 comprises an absorption layer 11 and a laminated edge 12. The absorption layer 11 is positioned inside the main body 10. The laminated edge 12 is arranged surrounding the main body 10. The laminated edge arranged on each side of the main body 11 further respectively forms fixing wings 13.

The raised flow-guiding element 20 is positioned on the top of the absorption layer 11 of the main body 10. The raised flow-guiding element 20 comprises an arch-shaped flow-guiding layer 21, a front sheet 22, and a back sheet 23. Each of the two lateral sides of the flow-guiding layer 21 is respectively and intermittently connected to the main body 10. The maximum distance between a leading edge of the flow-guiding layer 21 and the main body 10 is smaller than the maximum distance between a trailing edge of the flow-guiding layer 21 and the main body 10. Multiple perforations 24 are formed on the top of the flow-guiding layer 21. The front sheet 22 is extended from the leading edge of the flow-guiding layer 21, and a leading edge of the front sheet 22 is connected to the main body 10. The two lateral sides of the front sheet 22 are not connected to the main body 10. The back sheet 23 is extended from the trailing edge of the flow-guiding layer 21, and the back sheet 23 is extended beyond the laminated edge 12 of the main body 10. A top margin of the flow-guiding layer 21 is tilted. The distance between the top margin of the flow-guiding layer 21 and the main body 10 increases progressively from a leading edge of the flow-guiding layer 21 to a trailing edge of the flow-guiding layer 21.

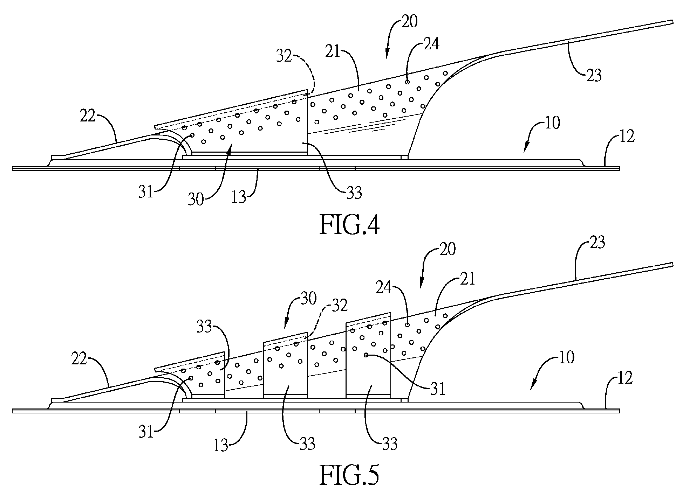

With references to Figs. 4 and 5, a second embodiment and a third embodiment of the sanitary pad in accordance with the present invention are similar to the first embodiment. The differences between the first embodiment and the second embodiment as well as the third embodiment include that the sanitary pad further comprises a ventilation unit 30. The ventilation unit 30 covers the flow-guiding layer 21 of the raised flow-guiding element 20. The ventilation unit 30 comprises multiple holes 31, and an interspace 32 is formed between the ventilation unit 30 and the flow-guiding layer 21 of the raised flow-guiding element 20. The interspace 32 is connected to each of the multiple holes 31 of the ventilation unit 30 and each of the multiple perforations 24 of the raised flow-guiding element 20.

With reference to Fig. 4, the ventilation unit 30 comprises a sheet 33. Each of the two lateral sides of the sheet 33 is respectively connected to each of the two lateral sides of the flow-guiding layer 21. The sheet 33 is positioned adjacent to the leading edge of the flow-guiding layer 21. Each of the multiple holes 31 is distributed on the sheet 33. With reference to Fig. 5, the ventilation unit 30 comprises multiple sheets 33. Each of the multiple sheets 33 is positioned intermittently on the raised flow-guiding element 20. Each of the two lateral sides of each of the sheets 33 is respectively connected to each of the two lateral sides of the flow-guiding layer 21. Each of the multiple holes 31 is distributed on each of the multiple sheets 33.

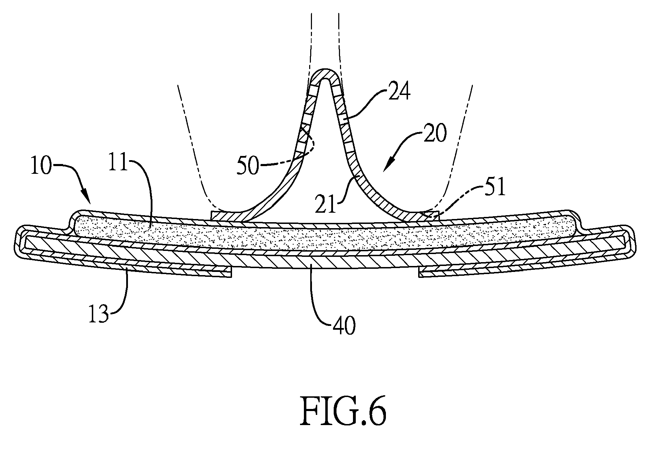

With reference to Fig 6, as a user uses the sanitary pad in accordance with the present invention, a surface of the sanitary pad with an adhesive opposite to the main body 10 can be adhered to the user's underpants 40. The user can pull the back sheet 23 of the raised flow-guiding element 20, allowing a top of the flow-guiding layer 21 and the front sheet 22 of the raised flow-guiding element 20 to be inserted into and adhered to the user's labia minora 50, and allowing the flow-guiding layer 21 adjacent to the absorption layer 11 of the main body 10 to be adhered to the user's labia majora 51. The menstrual blood flowing to the labia minora 50 can be absorbed directly and quickly by the flow-guiding layer 21 and the front sheet 22 of the raised flow-guiding element 20, preventing the menstrual blood from overflowing from the user's perineum and intergluteal cleft.

Besides, the multiple perforations 24 formed on the top of the flow-guiding layer 21, the multiple holes 31 distributed on the ventilation unit 30, and the interspace 32 formed between the ventilation unit 30 and the flow-guiding layer 21 enhance breathability and comfort. Moreover, the multiple perforations 24 formed on the top of the flow-guiding layer 21 enhance absorption efficiency of menstrual blood.

In a summary, the leading edge of the front sheet 22 is connected to the main body 10, and the maximum distance between a leading edge of the flow-guiding layer 21 and the main body 10 is smaller than the maximum distance between a trailing edge of the flow-guiding layer 21, allowing the flow-guiding element 20 to be formed as a raised structure with the distance between the flow-guiding layer 21 and the main body 10 progressively increasing from the leading edge of the flow-guiding layer 21 to the trailing edge of the flow-guiding layer 21. Therefore, the top of the flow-guiding layer 21 and the front sheet 22 of the raised flow-guiding element 20 can be inserted into the user's labia minora 50 and adhered to the user's labia majora 51. The sanitary pad in accordance with the present invention provides good adhesion effect, which allows the menstrual blood flowing to the labia minora 50 to be absorbed directly and quickly by the raised flow-guiding element 20, and further guides the menstrual blood to the main body 10, rather than absorbs and guides the menstrual blood when the blood flows out of the labia minora 50. Therefore, the sanitary pad in accordance with the present invention prevents the menstrual blood from overflowing.

The multiple perforations 24 formed on the top of the flow-guiding layer 21, the multiple holes 31 distributed on the ventilation unit 30, and the interspace 32 formed between the ventilation unit 30 and the flow-guiding layer 21 enhance breathability. Besides, the back sheet 23 extending beyond the laminated edge 12 of the main body 10 is convenient for the user to pull and adjust, allowing the trailing edge of the flow-guiding layer 21 to be placed into the user's intergluteal cleft. Therefore, the multiple perforations 24 formed on the top and trailing edge of the flow-guiding layer 21 also enhance breathability of the sanitary pad in accordance with present invention as the trailing edge of the flow-guiding layer 21 of the sanitary pad is placed into the user's intergluteal cleft.

Even though numerous characteristics and advantages of the present invention have been set forth in the foregoing description, together with details of the structure and features of the invention, the disclosure is illustrative only. Changes may be made in the details, especially in matters of shape, size, and arrangement of parts within the principles of the invention to the full extent indicated by the broad general meaning of the terms in which the appended claims are expressed.

## Claims

1. A sanitary pad **characterized in that** the sanitary comprises:
a main body (10), comprising an absorption layer (11) and a laminated edge (12), wherein the laminated edge (12) is arranged surrounding the main body (10); and,
a raised flow-guiding element (20), wherein the raised flow-guiding element (20) is positioned on the top of the absorption layer (11) of the main body 10; the raised flow-guiding element (20) comprising
an arch-shaped flow-guiding layer (21) having two lateral sides, wherein each of the two lateral sides of the flow-guiding layer (21) is respectively and intermittently connected to the main body (10); the maximum distance between a leading edge of the flow-guiding layer (21) and the main body (10) is smaller than the maximum distance between a trailing edge of the flow-guiding layer (21) and the main body (10);
a front sheet (22); wherein the front sheet (22) is extended from the leading edge of the flow-guiding layer (21), and a leading edge of the front sheet (22) is connected to the main body (10); and
a back sheet (23), wherein the back sheet (23) is extended from the trailing edge of the flow-guiding layer (21), and the back sheet (23) is extended beyond the laminated edge (12) of the main body (10).

2. The sanitary pad as claimed in claim 1, wherein the sanitary pad further comprises a ventilation unit (30); the ventilation unit (30) comprises multiple holes (31); the ventilation unit (30) covers the flow-guiding layer (21), allowing an interspace (32) to be formed between the ventilation unit (30) and the flow-guiding layer (21) of the raised flow-guiding element (20); each of the multiple holes (31) is distributed on the ventilation unit (30), and the interspace (32) is connected to each of the multiple holes (31) of the ventilation unit (30) and each of the multiple perforations (24).

3. The sanitary pad as claimed in claim 2, wherein the ventilation unit (30) further comprises a sheet (33) having two lateral sides; each of the two lateral sides of the sheet (33) is respectively connected to each of the two lateral sides of the flow-guiding layer (21); the sheet (33) is positioned adjacent to the leading edge of the flow-guiding layer (21); each of the multiple holes (31) is distributed on the sheet (33) of the ventilation unit (30).

4. The sanitary pad as claimed in claim 2, wherein the ventilation unit (30) further comprises multiple sheets (33) each having two lateral sides; each of the multiple sheets (33) is positioned intermittently on the raised flow-guiding element (20); each of the two lateral sides of each of the sheets (33) is respectively connected to each of the two lateral sides of the flow-guiding layer (21); each of the multiple holes (31) is distributed on each of the multiple sheets (33).

5. The sanitary pad as claimed in claim 1, wherein two lateral sides of the front sheet (22) are not connected to the main body (10).

6. The sanitary pad as claimed in claim 1, wherein a top margin of the flow-guiding layer (21) is tilted; the distance between the top margin of the flow-guiding layer (21) and main body (10) progressively increases from the leading edge of the flow-guiding layer (21) to the trailing edge of the flow-guiding layer (21).

7. The sanitary pad as claimed in claim 5, wherein a top margin of the flow-guiding layer (21) is tilted; the distance between the top margin of the flow-guiding layer (21) and main body (10) progressively increases from the leading edge of the flow-guiding layer (21) to the trailing edge of the flow-guiding layer (21).
